# EUROPEAN PATENT SPECIFICATION

(11) **EP 3 115 350 B1**
(45) Date of publication and mention of the grant of the patent: **06.02.2019**
(21) Application number: 15175465.2
(22) Date of filing: 06.07.2015
(51) Int. Cl.: C07C 29/20, C07C 45/00, B01J 8/06

(54) **A PROCESS FOR THE PRODUCTION OF A MIXTURE COMPRISING CYCLOHEXANOL AND CYCLOHEXANONE**
VERFAHREN ZUR HERSTELLUNG EINES GEMISCHES MIT CYCLOHEXANOL UND CYCLOHEXANON
PROCÉDÉ POUR LA PRODUCTION D'UN MÉLANGE COMPRENANT DU CYCLOHEXANOL ET DU CYCLOHEXANONE

(43) Date of publication of application: 11.01.2017
(73) Proprietor: CAP III B.V., 6129 EL Urmond (NL)
(72) Inventor: MARTENS, Wilhelmus Rudolf Maria, 6129 EL Urmond (NL); TINGE, Johan Thomas, 6129 EL Urmond (NL)
(74) Representative: Cohausz & Florack

(56) References cited:
- WO-A1-2009/080621
- WO-A1-2011/073233
- US-A- 5 484 576

## Description

### Field of the invention

The present invention relates to a process for constructing a chemical plant, which is used for the production of a mixture comprising cyclohexanone and cyclohexanol by hydrogenation of phenol.

### Background of the invention

Cyclohexanone is an intermediate in the production of, amongst other compounds, adipic acid and caprolactam. These are monomers commonly used in the production of polyamide-6,6 and polyamide-6, respectively. A major process for the production of cyclohexanone for use in producing caprolactam is based on selective oxidation of cyclohexane, using atmospheric oxygen. Oxidation of cyclohexane yields a mixture of cyclohexanol and cyclohexanone and the precursor cyclohexyl hydroperoxide which is then thermally and/or catalytically decomposed to produce additional cyclohexanol and cyclohexanone, and a variety of by-products. Cyclohexanone may be separated by distillation from the mixture comprising cyclohexanol, cyclohexanone, unreacted cyclohexane and by-products. Cyclohexanol may also be recovered by distillation and optionally converted to cyclohexanone by dehydrogenation. Chemical processes for the production of cyclohexanone and cyclohexanol by oxidation of cyclohexane are known in the art (see for example Michael Tuttle Musser; Cyclohexanol and Cyclohexanone in Ullmann's Encyclopedia of Industrial Chemistry. Published Online: 15 OCT 2011 DOI: 10.1002/14356007.a08_217.pub2 Copyright © 2002 by Wiley-VCH Verlag GmbH & Co. KGaA) (Musser).

The ratio of cyclohexanone to cyclohexanol in the mixture obtained after decomposition of the precursor hydroxyl hydroperoxide that is obtained in the oxidation process of cyclohexane is in general from 0.3 to 2. The ratio depends, amongst others, on the presence, if any, and type and concentration of catalyst in the cyclohexane oxidation unit. This ratio determines to a large extent the capacity of the cyclohexanol dehydrogenation section. In all cases a large fraction of the cyclohexanone that is produced from cyclohexane is obtained by dehydrogenation of cyclohexanol.

Typically, cyclohexanone and hydrogen are produced from dehydrogenation of cyclohexanol. The process for the dehydrogenation of cyclohexanol to cyclohexanone can be described by the following stoichiometric equation:

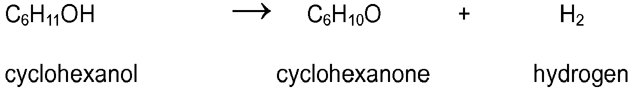

Generally, the dehydrogenation reaction of cyclohexanol to cyclohexanone is carried out in the vapour phase (also often called: gas phase) in the presence of catalytic material. In general, in such a dehydrogenation process a heterogeneous catalyst is used. Suitable catalysts include catalysts based on copper and/or zinc, for example catalysts comprising CuCrO₄2CuO.2H₂O, CuMgO, CuZnO, CuCrO, CuCrMnV and/or ZnO.

The dehydrogenation of cyclohexanol to cyclohexanone is known to be endothermic and is generally performed at enhanced temperatures. Present industrial processes for the production of cyclohexanone through the catalytic dehydrogenation of cyclohexanol differ in the heat carrier for the introduction of the heat of reaction. In general heating is done with flue gases, thermic oil, liquid metals or steam as heat carrier. Often, this heating is done with steam or thermic oil as heat carrier, most often with steam.

The dehydrogenation of cyclohexanol to cyclohexanone may be carried out in any type of suitable reactor. In particular, the reactor may be selected from packed bed reactors, slurry reactors and shell and tube heat exchange reactors. Most preferably, the dehydrogenation is carried out in a shell and tube heat exchange reactor with dehydrogenation catalyst in the tubes and the heating medium outside the tubes. Most preferably, both the feed and the discharge of the cyclohexanol dehydrogenation reactor are in the gaseous state. Typically, the cyclohexanol dehydrogenation reactor is a (vertical) multi-tubular catalytic reactor with catalytic material arranged in the tubes and the heat carrier circulating externally around the tubes. The reactor is typically fed with a gaseous mixture comprising cyclohexanol.

One alternative process for the production of cyclohexanone is by the selective hydrogenation of benzene to cyclohexene, followed by the hydration of the cyclohexene to cyclohexanol, which is then followed by dehydrogenation of the cyclohexanol to cyclohexanone. Some of these chemical steps are performed under harsh conditions regarding temperature and or pressure. This process is described in, for example, Musser. However, this process produces several undesired by-products that are difficult to remove. And in addition, this process is highly energy intensive and requires high investments.

In the hydration of cyclohexene to cyclohexanol, the reaction mixture exiting the hydration section contains almost no cyclohexanone. Accordingly, virtually all cyclohexanone that is produced is obtained by dehydrogenation of cyclohexanol. The dehydrogenation of cyclohexanol to cyclohexanone may be carried out in the same manner as described above.

Another alternative process for the production of cyclohexanone is by the catalytic reduction of phenol with hydrogen, for example using a palladium-comprising heterogeneous catalyst.

WO 2011/073233 describes a process for the production of cyclohexanone by hydrogenation of phenol in which the hydrogenation catalyst is treated with water by continuously or intermittently feeding water (or steam) during the hydrogenation reaction, resulting in an increased conversion of phenol.

Typically, a mixture comprising cyclohexanone and cyclohexanol is produced from the hydrogenation of phenol. The processes for the hydrogenation of phenol to cyclohexanone and cyclohexanol can be described by the following stoichiometric equations:
and

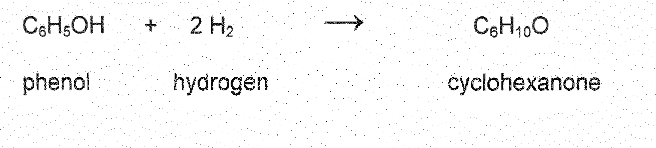

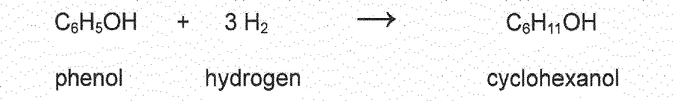

The hydrogenation of phenol with hydrogen can be performed in the vapour phase (also often called: gas phase) or in the liquid phase, described in, for example Musser; J.F. Van Peppen, W.B. Fisher and C.H. Chan, 'Phenol Hydrogenation Process' in Chemical Industries, 22 ('Catalysis of Organic Reactions'; Ed. R.L. Augustine); Marcel and Dekker, N.Y., 355-372', (1985); and A.C. Dimian and C.S. Bildea, Chemical Process Design, Computer-Aided Case Studies', Wiley-VCH Verlag GmbH & Co. KGaA, Weinheim, Germany, Chapter 5, 129-172 (2008). The hydrogenation of phenol is known to be highly exothermic. A mixture comprising cyclohexanol, cyclohexanone, unreacted phenol and by-products is produced. Separation of cyclohexanone from this mixture may be made by distillation. Cyclohexanol may also be recovered by distillation and optionally converted to

cyclohexanone by dehydrogenation. Unreacted phenol is removed by distillation and recycled in the process.

Typically, a phenol hydrogenation reactor is a (vertical) multi-tubular catalytic reactor with catalytic material in the tubes and the heat carrier circulates externally around the tubes, that is fed with a gaseous mixture comprising hydrogen and phenol. In this reactor the vast majority of the fed phenol is converted into cyclohexanone. Heat of reaction is removed by indirect cooling with a coolant, in general, the coolant is a liquid, which is optionally evaporated: in case liquid water is applied as coolant then steam is optionally obtained by evaporation of liquid water within the shell of the reactor. The reactor temperature is generally controlled to be in the range of from 100 to 250 °C.

The molar ratio of cyclohexanone to cyclohexanol in the reaction mixture exiting the phenol hydrogenation section is in general more than 5. So, in a process for the production of cyclohexanone based on catalytic reduction of phenol with hydrogen just a small fraction of the phenol is converted into cyclohexanol. In such a process the cyclohexanol might be removed and used for other applications, e.g. production of adipic acid. Alternatively, cyclohexanol produced may be dehydrogenated to yield cyclohexanone. Such a process may be carried out in the same manner as described above. However, processes for the production of cyclohexanone based on catalytic reduction of phenol with hydrogen do not require a cyclohexanol dehydrogenation section in order to produce an economically viable quantity of cyclohexanone. In case a process for the production of cyclohexanone based on catalytic reduction of phenol with hydrogen is provided with a dehydrogenation unit to convert cyclohexanol into cyclohexanone, then the required capacity of the cyclohexanol dehydrogenation section can be quite limited compared to the capacity of the process for the production of cyclohexanone, because in such a process just a small fraction of the produced cyclohexanone is obtained by dehydrogenation of cyclohexanol.

In the oxidation of cyclohexane, the produced mixture of cyclohexanol and cyclohexanone typically comprises a large proportion of cyclohexanone. In the hydration of cyclohexene, typically the produced mixture of cyclohexanol and cyclohexanone comprises almost entirely cyclohexanol. However, in the hydrogenation of phenol, the produced mixture of cyclohexanol and cyclohexanone comprises only a small minority of cyclohexanol. Accordingly, to produce a given volume of cyclohexanone, the process for the hydrogenation of phenol requires far less volume of cyclohexanol to be dehydrogenated to cyclohexanone than does either the process for the oxidation of cyclohexane or the process for the hydration of cyclohexene.

Chemical plants for the production of cyclohexanone by reduction of phenol, chemical plants for the production of cyclohexanone based on hydration of cyclohexene followed by dehydrogenation of cyclohexanol, and chemical plants for the production of cyclohexanone by oxidation of cyclohexane are all known in the art, also described in, for example, Musser. The starting materials, intermediates and by-products present in a plant for the production of cyclohexanone based on hydrogenation of phenol are different to those present in a plant for the production of cyclohexanone based on hydration of cyclohexene followed by dehydrogenation of cyclohexanol, and to those present in a plant for the production of cyclohexanone based on oxidation of cyclohexane. Accordingly, the apparatus required for the production of cyclohexanone based on hydrogenation of phenol is different to that required for the production of cyclohexanone based on hydration of cyclohexene followed by dehydrogenation of cyclohexanol, and also different to that required for the production of cyclohexanone based on oxidation of cyclohexane.

Nowadays, due to severe reduction of the phenol price compared to the benzene price, processes for the production of a mixture comprising cyclohexanone and cyclohexanol by reduction of phenol with hydrogen are more economic regarding raw materials costs and energy costs than both the process for producing cyclohexanone based on hydration of cyclohexene followed by dehydrogenation of cyclohexanol, and the process for producing cyclohexanone based on oxidation of cyclohexane. In addition the process conditions for the production of cyclohexanone by hydrogenation of phenol are much less harsh, especially regarding pressure, than the process conditions for the production of cyclohexanone based on hydration of cyclohexene followed by dehydrogenation of cyclohexanol, and the process conditions for the production of cyclohexanone by the oxidation of cyclohexane.

For current producers of a mixture comprising cyclohexanone that apply a process of hydration of cyclohexene followed by dehydrogenation of cyclohexanol, or for producers of a mixture comprising cyclohexanone by a process of oxidation of cyclohexane it is advantageous to switch to the process for the production of a mixture comprising cyclohexanone and cyclohexanol by hydrogenation of phenol. However, there arises a problem in that neither their current dedicated plant for the production of a mixture comprising cyclohexanone by hydration of cyclohexene followed by dehydrogenation of cyclohexanol; nor their current dedicated plant for the production of a mixture comprising cyclohexanone by the oxidation of cyclohexane are suitable for carrying out a process for the production of a mixture of cyclohexanone and cyclohexanol by the reduction of phenol. An option for switching to the process based on the hydrogenation of phenol is to build a fully new chemical plant. A major drawback of this approach is the high investment cost. In particular a reactor suitable for the hydrogenation of phenol is typically complex and therefore an expensive component.

The present inventors have discovered a method to significantly reduce the investment cost of a plant for the production of a mixture comprising cyclohexanone and cyclohexanol based on the reduction of phenol. They have developed a process for the production of a mixture comprising cyclohexanone and cyclohexanol by hydrogenation of phenol; based on the equipment of a chemical plant used to produce a mixture comprising cyclohexanone by oxidation of cyclohexane followed by dehydrogenation of cyclohexanol, or based on the equipment of a plant used to produce a mixture comprising cyclohexanone by hydration of cyclohexene followed by dehydrogenation of cyclohexanol. More specifically, the present invention provides a process for the construction of a second chemical plant, which second chemical plant is used for the production of a mixture comprising cyclohexanone and cyclohexanol by hydrogenation of phenol, said process comprising:
a) disconnecting a multi-tubular reactor that has been used for producing cyclohexanone by dehydrogenation of cyclohexanol from a first chemical plant, which first chemical plant was used for the production of a mixture comprising cyclohexanone by either:
   i) oxidation of cyclohexane followed by dehydrogenation of cyclohexanol; or
   ii) hydration of cyclohexene followed by dehydrogenation of cyclohexanol;
b) replacing the cyclohexanol dehydrogenation catalyst inside the tubes of said multi-tubular reactor with phenol hydrogenation catalyst; and
c) connecting said multi-tubular reactor to said second chemical plant; wherein "has been used" means that the multi-tubular reactor was
designed for the dehydrogenation of cyclohexanol and installed in a chemical plant for the dehydrogenation of cyclohexanol.

Typically, in the process of the present invention the multi-tubular reactor that has been used for the dehydrogenation of cyclohexanol was part of a chemical plant, which chemical plant was used to produce a mixture comprising cyclohexanone by oxidation of cyclohexane followed by dehydrogenation of cyclohexanol.

Typically, in the process of the present invention the multi-tubular reactor that has been used for the dehydrogenation of cyclohexanol was part of a chemical plant, which chemical plant was used to produce a mixture comprising cyclohexanone by hydration of cyclohexene followed by dehydrogenation of cyclohexanol.

As used herein, "has been used" includes that the multi-tubular reactor was designed for the dehydrogenation of cyclohexanol and/or installed in a chemical plant for the dehydrogenation of cyclohexanol.

A multi-tubular catalytic reactor that is used for the dehydrogenation of cyclohexanol in either a process of hydration of cyclohexene followed by dehydrogenation of cyclohexanol; or in a process of oxidation of cyclohexane followed by dehydrogenation of cyclohexanol has a large capacity because a large fraction of the produced cyclohexanone is obtained by dehydrogenation of cyclohexanol. Such a multi-tubular catalytic reactor is an expensive piece of equipment. Re-use of such a reactor in a process for the production of a mixture comprising cyclohexanone and cyclohexanol by hydrogenation of phenol will reduce the investment cost of such a process.

Both processes for the production of essentially pure cyclohexanone based on oxidation of cyclohexane followed by dehydrogenation of cyclohexanol and processes for the production of essentially pure cyclohexanone based on hydration of cyclohexene followed by dehydrogenation of cyclohexanol are known to consume large amounts of energy, typically supplied industrially as steam and electricity. Specific steam consumptions of more than 5 tons of steam per ton of purified cyclohexanone are known. Large quantities of steam are consumed in the various reaction sections, the removal of unreacted components and by-products, the distillative purification of cyclohexanone and the dehydrogenation of cyclohexanol. A further advantage of a process of constructing a plant to carry out a process for the production of a mixture of cyclohexanol and cyclohexanone according to the present invention is that the energy consumption per unit weight of cyclohexanone produced may be reduced. Typical specific steam consumption of hydrogenation of phenol to cyclohexanone is less than 2 tons of steam per ton of purified cyclohexanone and in general even less than 1 ton of steam per ton of purified cyclohexanone.

An additional advantage of the current invention is that the process for the production of a mixture of cyclohexanol and cyclohexanone based on hydrogenation of phenol requires much less harsh process conditions than either a process based on oxidation of cyclohexane followed by dehydrogenation of cyclohexanol or a process based on hydration of cyclohexene followed by dehydrogenation of cyclohexanol.

Typical carbon efficiency of hydrogenation of phenol to cyclohexanone is higher than 98 % and in general even higher than 99 %, while the carbon efficiency of oxidation of cyclohexane to cyclohexanone, including dehydrogenation of cyclohexanol and purification, is typically from 75 % to 90 % and the carbon efficiency of hydration of cyclohexene followed by dehydrogenation of cyclohexanol is typically from 92 % to 97 %. A yet further advantage of constructing a plant according to the present invention is that less starting material is required to produce a certain amount of cyclohexanone. Further, the amount of by-products and therefore waste produced per unit weight of cyclohexanone produced may be reduced. The production of cyclohexanone by the oxidation of cyclohexane is typically subject to stringent safety regulations because of the risk of ignition of explosive cyclohexane-oxygen mixtures. Yet a further advantage of the process of the present invention is that the risk of explosion of cyclohexane-oxygen mixtures is avoided, because no cyclohexane is used in the process of the constructed plant. Thus the associated safety measures are not required.

Also described herein is a chemical plant suitable for the production of a mixture comprising cyclohexanone and cyclohexanol by hydrogenation of phenol in a multi-tubular reactor, characterised in that said multi-tubular reactor has been used for the dehydrogenation of cyclohexanol.

Typically, in the process of the present invention said chemical plant for the hydrogenation of phenol further comprises one or more of the following units: an evaporator unit suitable for evaporating phenol, a partial condenser unit suitable for condensing cyclohexanol and cyclohexanone from the gaseous mixture comprising cyclohexanone and cyclohexanol, a distillation column suitable for distilling overhead components having a lower boiling point than cyclohexanone, a distillation column suitable for the separation of cyclohexanone with a purity of more than 99 wt. %, a distillation column suitable for distilling overhead components having a higher boiling point than cyclohexanone, a distillation column suitable for distilling as bottom product components having a higher boiling point than phenol, and a cyclohexanol hydrogenation reactor, wherein cyclohexanol is converted into cyclohexanone and hydrogen.

Typically, the chemical plant of the present invention further comprises a distillation column suitable for the separation of cyclohexanone with a purity of more than 99 wt.% from a mixture comprising cyclohexanone and cyclohexanol.

Further described herein is cyclohexanone prepared in a chemical plant, in which chemical plant a mixture comprising cyclohexanone and cyclohexanol is produced in a multi-tubular reactor, characterised in that said multi-tubular reactor has been used for the dehydrogenation of cyclohexanol. Preferably, the mixture comprising cyclohexanone and cyclohexanol is produced by a continuous process for the hydrogenation of phenol.

A chemical plant includes all apparatus necessary to manufacture or otherwise process the products desired. This includes units for one or multiple chemical or physical operations, for example, heating up, cooling down, mixing, distillation, extraction and reaction. It includes all auxiliary equipment, for example reflux units, coolant supply, pumps, heat exchangers and pipework. The exact apparatus depends amongst others on type and purity of the starting material(s) and the desired end product(s), but also on the scale and type of process.

By a continuous process for the hydrogenation of phenol is meant a process in which phenol and hydrogen are fed without interruption and whereby a hydrogenated product or a mixture of hydrogenated products of phenol are withdrawn without interruption. A continuous process for the hydrogenation of phenol may be at a constant rate or may fluctuate in rate over time. A continuous process for the hydrogenation of phenol may be interrupted for a certain period of time due to e.g. a process disturbance, a maintenance activity, or for economic reasons.

The chemical plant is preferably of industrial scale. By industrial scale is meant a hydrogenation rate of phenol of at least 1,000 kg of phenol per hour, more preferably at least 2,000 kg of phenol per hour, even more preferably at least 4,000 kg of phenol per hour, and most preferably at least 6,000 kg of phenol per hour.

By hydrogenation of phenol is meant that phenol and hydrogen are partly or completely converted and whereby a hydrogenated product or a mixture of hydrogenated products of phenol are formed. Preferably, at least 50 mole % of the converted phenol is converted into either cyclohexanone or a mixture of cyclohexanone and cyclohexanol. More preferably at least 70 mole %; even more preferably at least 90 mole %; at least 95 mole %; especially at least 98 mole % of the converted phenol is converted into either cyclohexanone or a mixture of cyclohexanone and cyclohexanol.

As used herein, a mixture comprising cyclohexanone and cyclohexanol that is produced in a process for the hydrogenation of phenol is the mixture of compounds resulting from the hydrogenation of phenol. Typically, this comprises cyclohexanone, cyclohexanol, at least one characteristic by-product and (unreacted) phenol. The phenol content of this mixture is typically at least 0.02 wt.%. Preferably, it is at least 0.03 wt.%; more preferably at least 0.1 wt.%. The phenol content is preferably less than 50 wt.%; more preferably less than 20 wt.%; most preferably less than 10 wt.%. The mixture is typically gaseous.

Typically, the mixture comprising cyclohexanone and cyclohexanol that is produced comprises one or more of the following components: phenol, 2-phenylcyclohexanol, 3-phenylcyclohexanol, 4-phenylcyclohexanol, cyclohexylphenylether, benzofuran, 2,3-dimethylbenzofuran, 3-methyl-4-octanone, 4-methyl-3-octanone, 3-methyl-3-octanone, methyl-isopropylcyclohexanol, methyl-isopropylcyclohexanone and 1-(4-methylpentane-2-yl)-benzene-phenol. In addition, the gaseous mixture comprising cyclohexanone and cyclohexanol that is produced might also comprise (unconverted) hydrogen and inert components like nitrogen and methane.

Typically, the mixture comprising cyclohexanone and cyclohexanol that is produced also comprises phenol and at least one compound selected from 2-phenylcyclohexanol, 3-phenylcyclohexanol, 4-phenylcyclohexanol, cyclohexylphenylether, benzofuran, 2,3-dimethylbenzofuran, 3-methyl-4-octanone, 4-methyl-3-octanone, 3-methyl-3-octanone, methyl-isopropylcyclohexanol, methyl-isopropylcyclohexanone and 1-(4-methylpentane-2-yl)-benzene-phenol.

Typically, a mixture comprising phenol and hydrogen is charged to the multi-tubular reactor. Typically, the molar ratio of hydrogen to phenol in the mixture comprising phenol and hydrogen that is charged to the multi-tubular reactor is more than 1.5. Preferably, the molar ratio of hydrogen to phenol is more than 1.8; yet more preferably, the molar ratio is more than 2; even more preferably, the molar ratio is more than 3. The molar ratio of hydrogen to phenol in the mixture comprising phenol and hydrogen that is charged as feed to the multi-tubular reactor is less than 15. Preferably, the molar ratio of hydrogen to phenol is less than 10; yet more preferably, the molar ratio is less than 7; even more preferably, the molar ratio is less than 5.

Typically, the molar ratio of cyclohexanone to cyclohexanol in the mixture comprising cyclohexanone and cyclohexanol that is discharged from the multi-tubular reactor is more than 4. Preferably, the molar ratio of cyclohexanone to cyclohexanol in the mixture comprising cyclohexanone and cyclohexanol that is discharged is more than 6. More preferably, the molar ratio is more than 10; yet more preferably, the molar ratio is more than 15.

Typically, a mixture comprising phenol and hydrogen is charged to the multi-tubular reactor; wherein the molar ratio of hydrogen to phenol in the said mixture comprising phenol and hydrogen that is charged as feed to the multi-tubular reactor is more than 1.5 and wherein the molar ratio of cyclohexanone to cyclohexanol in the mixture comprising cyclohexanone and cyclohexanol that is discharged from the multi-tubular reactor is more than 4.

Typically, cyclohexanone is separated from the mixture comprising cyclohexanone and cyclohexanol that is produced. More preferably, cyclohexanone with a purity of more than 90 wt.% is separated; even more preferably, cyclohexanone with a purity of more than 98 wt.% is separated. Typically, the separation of cyclohexanone is performed by distillation, crystallization, extraction and/or a combination thereof.

Typically, from the mixture comprising cyclohexanone and cyclohexanol that is produced, cyclohexanone with a purity of more than 99 wt.% is separated by distillation.

Typically, the hydrogenation of phenol is performed in the gas phase. The number of reactor tubes in the multi-tubular reactor is typically more than 5. Preferably, it is more than 10. More preferably, it is more than 25. The number of reactor tubes is typically less than 50,000. Preferably, it is less than 40,000. More preferably, it is less than 20,000. Typically, in the process of the present invention the number of reactor tubes in said multi-tubular reactor is from 25 to 20,000.

The internal diameter of the shell of the multi-tubular reactor is typically more than 50 mm. Preferably, it is more than 100 mm. More preferably, it is more than 200 mm. The internal diameter of the shell of the multi-tubular reactor is typically less than 10 m. Preferably, it is less than 8 m. More preferably, it is less than 6 m. Typically, in the process of the present invention the internal diameter of the shell of said multi-tubular reactor is from 0.2 to 8 m.

The internal diameter of the reactor tubes is typically more than 2 mm. Preferably, it is more than 5 mm. More preferably, it is more than 10 mm. The internal diameter of the reactor tubes is typically less than 500 mm. Preferably, it is less than 250 mm. More preferably, it is less than 120 mm.

Typically, in the process of the present invention the number of reactor tubes in said multi-tubular reactor is from 25 to 20,000, and the internal diameter of each of the reactor tubes in said multi-tubular reactor is from 10 to 120 mm. Preferably, all reactor tubes in the multi-tubular reactor have (almost) the same internal diameter.

In the tubes in the multi-tubular reactor heat is generated due to hydrogenation of phenol, thereby heating up the mixture of components in the tubes. Heat of the gaseous components is removed by indirect cooling with a coolant. The temperature of the mixture comprising cyclohexanone and cyclohexanol that is discharged from the multi-tubular reactor is typically more than 60 °C. Preferably, it is more than 80 °C. More preferably, it is more than 100 °C. The temperature of the mixture comprising cyclohexanone and cyclohexanol that is discharged from the multi-tubular reactor is typically less than 260 °C. Preferably, it is less than 240 °C. More preferably, it is less than 220 °C. Typically, the mixture comprising cyclohexanone and cyclohexanol that is discharged from the multi-tubular reactor is in the range of from 100 to 220° C.

The pressure of the mixture comprising cyclohexanone and cyclohexanol that is charged to the multi-tubular reactor is typically more than 0.01 MPa. Preferably, it is more than 0.05 MPa. More preferably, it is more than 0.1 MPa. The pressure of the mixture comprising cyclohexanone and cyclohexanol that is charged to the multi-tubular reactor is typically less than 5 MPa. Preferably, it is less than 3 MPa. More preferably, it is less than 1 MPa. Typically, the pressure at the point of discharge of the mixture comprising cyclohexanone and cyclohexanol from the multi-tubular reactor is from 0.1 to 1.0 MPa.

Typically, in a multi-tubular reactor wherein phenol is hydrogenated heat is removed by a coolant. Usually, as coolant aqueous or organic solvents or mixtures thereof are applied. Typically, water is applied as coolant in the multi-tubular reactor. By absorbing heat the coolant that is charged to the multi-tubular reactor is heated up, evaporated (partly or completely) or a combination thereof. Preferably, at least 10 wt.% of the coolant that is charged to the multi-tubular reactor is evaporated. More preferably, at least 50 wt.%. Even more preferably, at least 90 wt.%. Typically, in the process of the present invention water is applied as coolant in the multi-tubular reactor and more than 90 wt.% of the water is evaporated.

The hydrogenation of phenol with hydrogen can be performed in the gas phase or in the liquid phase. Preferably, the hydrogenation of phenol with hydrogen is performed in the gas phase. Typically, a process for the hydrogenation of phenol that is performed in the gas phase is charged with a gaseous mixture comprising the raw materials phenol and hydrogen as feed. Preferably, the phenol content of the mixture comprising the raw materials phenol and hydrogen that is charged as feed to the multi-tubular reactor is at least 5 wt. %. More preferably, at least 15 wt.%. Even more preferably, at least 25 wt.%. Typically, in the process of the present invention a gaseous mixture comprising phenol and hydrogen, in which the phenol content is more than 25 wt. %, is charged as feed to the multi-tubular reactor.

The hydrogenation catalyst may in principle be any (supported) hydrogenation catalyst capable of catalysing the hydrogenation of phenol. Usually, the catalyst comprises one or more catalytically active metals selected from palladium, platinum, ruthenium, rhodium, iridium, rubidium and osmium. Palladium, platinum or a combination thereof are preferred catalytically active metals.

Typically, the hydrogenation is carried out in the presence of a palladium comprising catalyst.

The support may in principle be any support capable of supporting one or more catalytically active metals for the hydrogenation of phenol. Suitable supports in particular may be selected from the group of alumina, activated carbon, titanium oxide, calcium carbonate and carbon black. Another support that may be used is silica.

Typically, the hydrogenation is carried out in the presence of a supported palladium-comprising catalyst.

In general, the per pass conversion of phenol in the reaction unit is more than 90 %. Optionally, unreacted hydrogen gas and inerts are separated off from the reaction mixture. Usually, unreacted hydrogen gas is re-used in the phenol hydrogenation process.

As used herein, the meaning of an "essentially pure" component is that the content of the component is at least 98 wt.%. Preferably, it is at least 99 wt.%; more preferably at least 99.5 wt.%; even more preferably at least 99.9 wt.%.
FIG. 1 shows a plant for the production of cyclohexanone based on oxidation of cyclohexane followed by dehydrogenation of cyclohexanol.
FIG. 2 shows a plant for the production of cyclohexanone based on hydration of cyclohexene followed by dehydrogenation of cyclohexanol.
FIG. 3 shows a plant according to the present invention, for the production of cyclohexanone by hydrogenation of phenol.

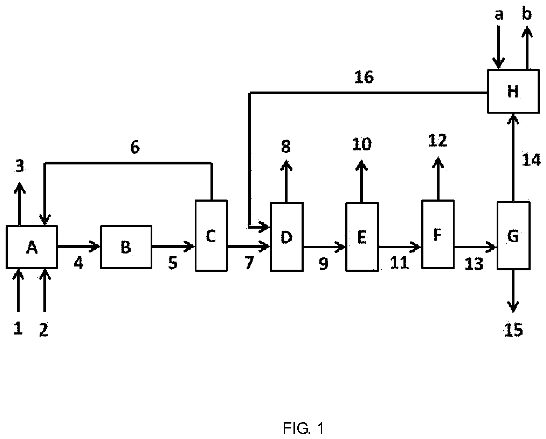

FIG. 1 shows a plant for the production of cyclohexanone by first oxidising cyclohexane and then separating cyclohexanone from the resulting mixture and finally dehydrogenating cyclohexanol into cyclohexanone. The cyclohexanol dehydrogenation is carried out in a one or one or more multi-tubular reactors.

Cyclohexane oxidation unit [A] comprises one or more oxidation reactors. Cyclohexane is fed to cyclohexane oxidation unit [A] through line [1]. Air is fed to cyclohexane oxidation unit [A] through line [2]. Off-gases exit cyclohexane oxidation unit [A] through line [3] and are charged to a heat recovery unit (not shown in FIG. 1). The resulting oxidised mixture which comprises cyclohexylhydroperoxide is fed through line [4] to cyclohexylhydroperoxide decomposition unit [B], where cyclohexylhydroperoxide is decomposed into cyclohexanone and/or cyclohexanol. Cyclohexylhydroperoxide decomposition unit [B] comprises one or more cyclohexylhydroperoxide decomposition reactors. Feeding of an aqueous sodium hydroxide solution and a catalyst to, and removal of an aqueous sodium hydroxide comprising solution from cyclohexylhydroperoxide decomposition unit [B] are not shown in FIG. 1. A resulting decomposed mixture is removed through line [5] to cyclohexane recovery unit [C]. Cyclohexane is distilled overhead in cyclohexane recovery unit [C] and is recycled to cyclohexane oxidation unit [A] through line [6]. Cyclohexane recovery unit [C] comprises one or more cyclohexane distillation columns. The bottom product comprising a mixture of cyclohexanol and cyclohexanone is discharged through line [7]. Optionally, this bottom product comprising a mixture of cyclohexanol and cyclohexanone is treated with an aqueous sodium hydroxide solution and/or washed with water (not shown in FIG. 1). The optionally treated bottom product is fed to first lights distillation column [D], where a first mixture of components with boiling points below that of cyclohexanone is distilled overhead through line [8]. The bottom product of first lights distillation column [D] is fed through line [9] to second lights distillation column [E], where a second mixture of components with boiling points below that of cyclohexanone is distilled overhead and removed through line [10]. The bottom product of second lights distillation column [E] is fed through line [11] to cyclohexanone distillation column [F], where essentially pure cyclohexanone is distilled overhead through line [12]. The bottom product of cyclohexanone distillation column [F] is fed through line [13] to cyclohexanol distillation column [G], where a mixture comprising cyclohexanol and cyclohexanone is distilled overhead. The bottom product of cyclohexanol distillation column [G] is removed through line [15]. The mixture comprising cyclohexanol and cyclohexanone is passed through line [14] to cyclohexanol dehydrogenation unit [H]. Cyclohexanol dehydrogenation unit [H] comprises one or more multi-tubular cyclohexanol dehydrogenation reactors with cyclohexanol dehydrogenation catalyst inside the tubes and with indirect heating with a heat transfer medium. The heating medium is fed to cyclohexanol dehydrogenation unit [H] through line [a] and is after transfer of heat discharged through line [b]. The resulting dehydrogenated mixture comprising cyclohexanone is, after separating of hydrogen gas (not shown in FIG. 1), recycled through line [16] to the first lights distillation column [D]. Optionally, the resulting dehydrogenated mixture comprising cyclohexanone is, after separating of hydrogen gas (not shown in FIG. 1), recycled through line [16] to second lights distillation column [E] (not shown in FIG. 1).

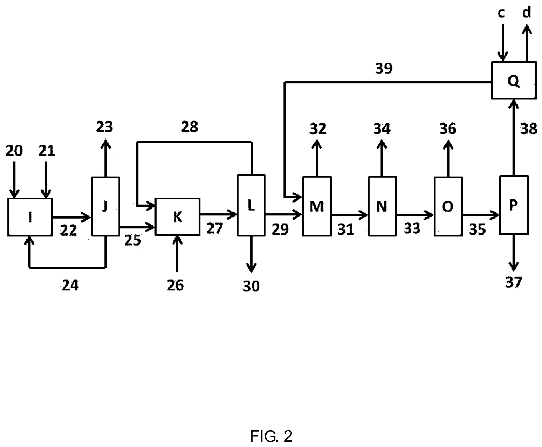

FIG. 2 shows a plant for the production of cyclohexanone based on hydrogenation of benzene into cyclohexene, followed by hydration of cyclohexene into cyclohexanol and followed by dehydrogenation of cyclohexanol into cyclohexanone.

Fresh benzene is fed through line [20], hydrogen gas is fed through line [21] and a mixture comprising benzene that is obtained in cyclohexene recovery section [J] is fed through line [24] to hydrogenation unit [I]. In hydrogenation unit [I] benzene is catalytically hydrogenated, whereby a mixture comprising benzene, cyclohexene and cyclohexane is produced. This mixture comprising benzene, cyclohexene and cyclohexane is fed to cyclohexene recovery section [J] through line [22]. From cyclohexene recovery section [J] a flow comprising cyclohexane is discharged through line [23], a flow comprising mainly cyclohexene is discharged through line [25], and a mixture comprising benzene is discharged through line [24]. In general, cyclohexene recovery section [J] comprises multiple extraction and/or distillation units (not shown in FIG. 2). The flow comprising mainly cyclohexene that is discharged through line [25] is fed to cyclohexene hydration unit [K]. Water is charged to cyclohexene hydration unit [K] though line [26]. A cyclohexanol-comprising product flow is discharged from cyclohexene hydration unit [K] though line [27] and is fed to distillation section [L]. From distillation section [L] a flow comprising cyclohexene is discharged through line [28] and is fed to cyclohexene hydration unit [K]; a flow comprising mainly cyclohexanol is discharged through line [29] and is fed to first lights distillation column [M]; and a flow comprising by-products is discharged through line [30]. Optionally, the flow comprising mainly cyclohexanol that is discharged through line [29] is fed to cyclohexanol dehydrogenation unit [Q] (not shown in FIG. 2). In the first lights distillation column [M] a first mixture of components with boiling points below that of cyclohexanone is distilled overhead through line [32]. The bottom product is discharged through line [31] and is fed to second lights distillation column [N], where a second mixture of components with boiling points below that of cyclohexanone is distilled overhead and removed through line [34]. The bottom product is discharged through line [33] and is fed to cyclohexanone distillation column [O], where essentially pure cyclohexanone is distilled overhead through line [36]. The bottom product of cyclohexanone distillation column [O] is discharged through line [35] and is fed to cyclohexanol distillation column [P], where a mixture comprising cyclohexanol and cyclohexanone is distilled overhead. The bottom product of cyclohexanol distillation column [P] is discharged through line [37]. The mixture comprising cyclohexanol and cyclohexanone is passed through line [38] to cyclohexanol dehydrogenation unit [Q]. Cyclohexanol dehydrogenation unit [Q] comprises one or more multi-tubular cyclohexanol dehydrogenation reactors with cyclohexanol dehydrogenation catalyst inside the tubes and with indirect heating with a heat transfer medium. The resulting dehydrogenated mixture comprising cyclohexanone is, after separating of hydrogen gas (not shown in FIG. 2), recycled through line [39] to first lights distillation column [M]. Optionally, the resulting dehydrogenated mixture comprising cyclohexanone is, after separating of hydrogen gas (not shown in FIG. 2), recycled through line [39] to second lights distillation column [N] (not shown in FIG. 2). The heating medium is fed to cyclohexanol dehydrogenation unit [Q] through line [c] and is after transfer of heat discharged through line [d].

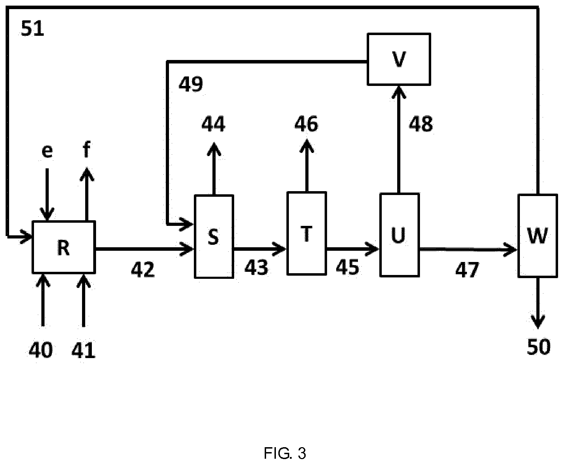

FIG. 3 shows a plant according to the present invention, for the production of cyclohexanone by first hydrogenating phenol in the vapour phase, then separating cyclohexanone from the resulting mixture comprising cyclohexanol and cyclohexanone and finally dehydrogenating cyclohexanol into cyclohexanone.

Fresh phenol is fed through line [40] and hydrogen gas is fed through line [41] and a mixture comprising phenol that is distilled overhead in phenol distillation column [W] is fed through line [51] to phenol hydrogenation unit [R]. Phenol hydrogenation unit [R] comprises one or more multi-tubular reactors with phenol hydrogenation catalyst inside the tubes and with indirect cooling with a coolant. Coolant is fed through line [e] and after being heated discharged through line [f]. The resulting mixture of reaction products, comprising phenol, cyclohexanol and cyclohexanone is discharged and fed to lights distillation column [S] through line [42]. Optionally, unreacted hydrogen gas and inert gases are separated from this mixture (not shown in FIG. 3). In lights distillation column [S] a mixture of components with boiling points below that of cyclohexanone is distilled overhead and is discharged through line [44]. The bottom product of lights distillation column [S] is discharged and is fed to cyclohexanone distillation column [T] through line [43]. Essentially pure cyclohexanone is distilled overhead in cyclohexanone distillation column [T] through line [46]. The bottom product of cyclohexanone distillation column [T] is discharged and is fed to cyclohexanol distillation column [U] through line [45]. A mixture comprising cyclohexanol and cyclohexanone is distilled overhead in cyclohexanol distillation column [U] and is passed to cyclohexanol dehydrogenation unit [V] through line [48]. Cyclohexanol dehydrogenation unit [V] comprises one or more cyclohexanol dehydrogenation reactors. The resulting mixture comprising cyclohexanone is, after separating of hydrogen gas (not shown in FIG. 3), recycled through line [49] to lights distillation column [S]. Optionally, the hydrogen gas obtained after separation is charged to phenol hydrogenation unit [R] (not shown in FIG. 3). The phenol-comprising bottom product is discharged from cyclohexanol distillation column [U] and is charged to phenol distillation column [W] through line [47]. In phenol distillation column [W] a mixture comprising phenol is distilled overhead and is fed to phenol hydrogenation unit [R] through line [51].The bottom product is removed from phenol distillation column [W] through line [50].

Optionally, the mixture comprising cyclohexanol and cyclohexanone that is distilled overhead in cyclohexanol distillation column [U] and is passed through line [48] is not fed to cyclohexanol dehydrogenation unit [V], but is discharged from the plant (not shown in FIG. 3). In such a case a plant according to the present invention, for the production of cyclohexanone by first hydrogenating phenol, does not require cyclohexanol dehydrogenation unit [V] and line [49].

According to one embodiment of the present invention, a chemical plant according to FIG. 3 is constructed by using equipment from a chemical plant according to FIG. 1. The cyclohexanol dehydrogenation unit [H] from the chemical plant according to FIG. 1 comprises one or more multi-tubular cyclohexanol dehydrogenation reactors. At least one of these multi-tubular reactors is disconnected from the plant suitable for the production of a mixture comprising cyclohexanone by oxidation of cyclohexane. Then at least one of these multi-tubular reactors is re-used for the construction of the phenol hydrogenation unit [R] as part of the chemical plant suitable for the production of a mixture comprising cyclohexanone and cyclohexanol by hydrogenation of phenol according to FIG. 3. This/these multi-tubular reactor(s) is/are connected to input lines for charging a mixture comprising phenol and hydrogen as feed to said multi-tubular reactor(s). This/these multi-tubular reactor(s) is/are connected to output lines for discharging a mixture comprising cyclohexanone and cyclohexanol from said multi-tubular reactor(s). Lines for charging water as coolant and discharging after being heated as steam are connected to the multi-tubular reactor(s).

The cyclohexanol dehydrogenation catalyst inside the tubes of the one or more multi-tubular reactor(s) is replaced by phenol hydrogenation catalyst.

The present invention therefore provides a process for the construction of a second chemical plant, which second chemical plant was is used for the production of a mixture comprising cyclohexanone and cyclohexanol by hydrogenation of phenol, said process comprising:
a) disconnecting a multi-tubular reactor that has been used for producing cyclohexanone by dehydrogenation of cyclohexanol from a first chemical plant, which first chemical plant is was used for the production of a mixture comprising cyclohexanone by either:
   i) oxidation of cyclohexane followed by dehydrogenation of cyclohexanol; or
   ii) hydration of cyclohexene followed by dehydrogenation of cyclohexanol;
b) replacing the cyclohexanol dehydrogenation catalyst inside the tubes of said multi-tubular reactor with phenol hydrogenation catalyst; and
c) connecting said multi-tubular reactor to said second chemical plant;
wherein "has been used" means that the multi-tubular reactor was designed for the dehydrogenation of cyclohexanol and installed in a chemical plant for the dehydrogenation of cyclohexanol.

The present invention is illustrated by, but not intended to be limited to, the following examples.

### COMPARATIVE EXPERIMENT A

A continuous process for the production of cyclohexanone by oxidation of cyclohexane was performed in a chemical plant, comprising:
- a cyclohexane oxidation unit;
- a cyclohexylhydroperoxide decomposition unit;
- a cyclohexane recovery unit;
- a first lights distillation column;
- a second lights distillation column;

## Claims

1. A process for the construction of a second chemical plant, which second chemical plant is used for the production of a mixture comprising cyclohexanone and cyclohexanol by hydrogenation of phenol, said process comprising:
a) disconnecting a multi-tubular reactor that has been used for producing cyclohexanone by dehydrogenation of cyclohexanol from a first chemical plant, which first chemical plant was used for the production of a mixture comprising cyclohexanone by either:
i) oxidation of cyclohexane followed by dehydrogenation of cyclohexanol; or
ii) hydration of cyclohexene followed by dehydrogenation of cyclohexanol;
b) replacing the cyclohexanol dehydrogenation catalyst inside the tubes of said multi-tubular reactor with phenol hydrogenation catalyst; and
c) connecting said multi-tubular reactor to said second chemical plant;
wherein "has been used" means that the multi-tubular reactor was designed for the dehydrogenation of cyclohexanol and installed in a chemical plant for the dehydrogenation of cyclohexanol.

2. A process according to claim 1, wherein the internal diameter of the shell of said multi-tubular reactor is from 0.2 to 8 m.

3. A process according to claim 1 or 2, wherein the number of reactor tubes in said multi-tubular reactor is from 25 to 20,000, and wherein the internal diameter of each of the reactor tubes in said multi-tubular reactor is from 10 to 120 mm.

## Patentansprüche

1. Ein Verfahren zur Konstruktion einer zweiten Chemieanlage, wobei die zweite Chemieanlage für die Herstellung eines Cyclohexanon und Cyclohexanol umfassenden Gemischs durch die Hydrierung von Phenol verwendet wird, wobei dieses Verfahren umfasst:
a) Trennen eines mehrröhrigen Reaktors, der für die Herstellung von Cyclohexanon durch Dehydrieren von Cyclohexanol verwendet worden ist, von einer ersten Chemieanlage, wobei die erste Chemieanlage für die Herstellung eines Cyclohexanon umfassenden Gemischs durch entweder:
I) Oxidation von Cyclohexan mit anschließender Dehydrierung des Cyclohexanols; oder
II) Hydration von Cyclohexen mit anschließender Dehydrierung des Cyclohexanols;
verwendet wurde,
b) Austauschen des Katalysators der Cyclohexanoldehydrierung in den Rohren des mehrröhrigen Reaktors gegen einen Katalysator der Phenolhydrierung; und
c) Anschließen dieses mehrröhrigen Reaktors an die zweite Chemieanlage;
wobei "verwendet worden ist" bedeutet, dass der mehrröhrige Reaktor für die Dehydrierung des Cyclohexanols konstruiert und in einer Chemieanlage für die Dehydrierung von Cyclohexanol eingebaut war.

2. Ein Verfahren nach Anspruch 1, wobei der Innendurchmesser des Mantels des mehrröhrigen Reaktors 0,2 bis 8 m beträgt.

3. Ein Verfahren nach Anspruch 1 oder 2, wobei die Anzahl der Reaktorrohre in dem mehrröhrigen Reaktor 25 bis 20 000 beträgt und wobei der Innendurchmesser jedes der Reaktorrohre in diesem mehrröhrigen Reaktor 10 bis 120 mm beträgt.

## Revendications

1. Procédé pour la construction d'une deuxième installation chimique, la deuxième installation chimique étant utilisée pour la production d'un mélange comprenant la cyclohexanone et le cyclohexanol par hydrogénation du phénol, ledit procédé comprenant :
a) déconnexion d'un réacteur multitubulaire, qui a été utilisé pour la production de cyclohexanone par déshydrogénation de cyclohexanol, d'une première installation chimique, la première installation chimique ayant été utilisée pour la production d'un mélange comprenant la cyclohexanone par :
i) oxydation de cyclohexane suivie de la déshydrogénation de cyclohexanol ; ou
ii) hydratation de cyclohexène suivie de la déshydrogénation du cyclohexanol ;
b) remplacement du catalyseur de déshydrogénation du cyclohexanol à l'intérieur des tubes du réacteur multitubulaire par un catalyseur d'hydrogénation du phénol; et
c) connexion dudit réacteur multitubulaire à ladite deuxième installation chimique ;
où « ayant été utilisée » signifie que le réacteur multitubulaire a été conçu pour la déshydrogénation du cyclohexanol et installé dans une installation chimique pour la déshydrogénation de cyclohexanol.

2. Procédé selon la revendication 1, où le diamètre interne de la coque dudit réacteur multitubulaire se situe dans l'intervalle allant de 0,2 à 8 m.

3. Procédé selon la revendication 1 ou 2, où le nombre de tubes du réacteur multitubulaire se situe dans l'intervalle allant de 25 à 20 000, et où le diamètre interne de chaque tube dans le réacteur multitubulaire se situe dans l'intervalle allant de 10 à 120 mm.
